(19)

Europäisches
Patentamt
European
Patent Office
Office européen
des brevets

(11) **EP 4 241 571 A1**

(12) **EUROPEAN PATENT APPLICATION**
published in accordance with Art. 153(4) EPC

(43) Date of publication:
**13.09.2023 Bulletin 2023/37**

(21) Application number: **21889228.9**

(22) Date of filing: **04.11.2021**

(51) International Patent Classification (IPC):
$A23J\ 3/14^{(2006.01)}$     $A23J\ 3/34^{(2006.01)}$
$A23C\ 11/10^{(2021.01)}$     $A23L\ 11/60^{(2021.01)}$
$A23L\ 11/65^{(2021.01)}$     $A23L\ 2/66^{(2006.01)}$
$A23L\ 2/38^{(2021.01)}$

(52) Cooperative Patent Classification (CPC):
**A23C 11/10; A23J 3/14; A23J 3/16; A23J 3/34;
A23L 2/38; A23L 2/66; A23L 11/60; A23L 11/65**

(86) International application number:
**PCT/JP2021/040557**

(87) International publication number:
**WO 2022/097675 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.11.2020  CN 202011222558**

(71) Applicants:
• **Amano Enzyme Inc.
Nagoya-shi
Aichi 460-8630 (JP)**

• **Amano Enzyme Manufacturing (China), Ltd.
Suqian City, Jiangsu Province, 223700 (CN)**

(72) Inventors:
• **WANG, Panhui
Wuxi, Jiangsu 214122 (CN)**
• **ZHAO, Shaohui
Wuxi, Jiangsu 214122 (CN)**

(74) Representative: **Müller-Boré & Partner
Patentanwälte PartG mbB
Friedenheimer Brücke 21
80639 München (DE)**

(54) **METHOD FOR PRODUCING PROCESSED PLANT-BASED MILK HAVING INCREASED
DISPERSION STABILITY AND/OR SOLUBILITY**

(57)    The purpose of the present invention is to provide processing technology that can improve the dispersion stability and/or solubility of a plant-based milk. In the present invention, walnut milk and/or peanut milk is treated with protein deamidase and lipase, whereby the dispersion stability of the processed walnut milk and/or processed peanut milk that is obtained can be improved. Furthermore, oat milk is treated with protein deamidase and cyclodextrin glucanotransferase, whereby the dispersion stability of the processed oat milk that is obtained can be improved. In addition, a plant-based milk selected from the group consisting of soy milk, peanut milk, and coconut milk is treated with protein deamidase and cyclodextrin glucanotransferase, whereby the solubility of the processed plant-based milk that is obtained can be improved.

EP 4 241 571 A1

**Description**

TECHNICAL FIELD

The present invention relates to a production method for a processed product of a plant protein food and drink material and/or a plant protein food and drink, and specifically to a production method for processed plant milk. More specifically, the present invention relates to a processing technique of increasing the dispersion stability and/or solubility of plant milk such as oat milk, black bean milk, walnut milk, peanut milk, and coconut milk.

BACKGROUND ART

Beverages rich in nutrients such as proteins have been widely familiar to people since nutrients can be easily taken. On the other hand, on the basis of an increase in the number of vegetarian people, allergic problems, religious reasons, and the like in recent years, soy milk using soybeans rich in a plant protein as a raw material has been widely spread as an alternative to animal milk typified by cow milk.

Regarding a soybean protein, various modification treatments have been studied for the purpose of improving existing characteristics thereof, providing food products having new preference characteristics, and the like.

For example, Patent Document 1 (JP 2000-50887 A) describes that the yield of soybean protein from soybean powder is improved by subjecting the soybean powder to a protein deamidase treatment. Patent Document 2 (JP 2008-283900 A) describes that a polyglycerol fatty acid ester containing a fatty acid having 12 to 22 carbon atoms as a main constituent fatty acid is effective as a dispersion stabilizer for soy milk. Patent Document 3 (JP 2015-159765 A) describes that by subjecting soy milk to a deamidation treatment with a cation exchange resin and/or a phytic acid removal treatment with an anion exchange resin, precipitation hardly occurs with respect to a coagulant.

On the other hand, from the viewpoint of coping with further diversification of preference and the like, further options other than soy milk are required for food and beverages containing plant proteins.

Therefore, milk of grains such as oats and black beans, and milk of nuts such as coconut, peanut, and walnut have been developed.

Among these, oat milk has a characteristic different from other grain milk in that the oat milk is rich in lipids, $\beta$-glucans, and minerals in addition to proteins, and a high nutritional value of the oat milk has attracted attention. For example, Patent Document 4 (US 6,451,361 B1) describes that by treating an oat suspension with an $\alpha$ amylase and a $\beta$ amylase, the problem of high viscosity is solved, and an oat dispersion in which a protein and a $\beta$ glucan are maintained is obtained. Patent Document 5 (CN 101991163 A) describes that maltooligosaccharide is produced by a treatment using an $\alpha$ amylase, a $\beta$ amylase, and a transglucosidase to improve the prebiotic action of an oat beverage.

Black bean milk has attracted attention in that it contains a black bean skin component abundantly containing components having various physiological effects such as anthocyanin. Patent Document 6 (JP 2006-230297 A) describes that, for the purpose of suppressing astringency or bitterness derived from the skin portion of black beans, yellow soybean soy milk is blended with black bean soy milk so that the content mass ratio (A : B) of a black bean soy milk solid content A and a yellow soybean soy milk solid content B is 4 : 6 to 6 : 4, whereby a black bean soy milk beverage excellent in preference is obtained.

Walnut milk contains much more lipids than proteins. Patent Document 7 (WO 2019/104971 A1) describes that walnut milk having low fat, high protein and good mouth feel, and stability is obtained by separating and removing a part of fat and oil during a production process.

Coconut milk is characterized by its unique aroma, and thus may be treated for the purpose of improving the flavor. For example, Patent Document 8 (JP 2008-099676 A) describes that a flavor-improving effect is obtained by adding sucralose to coconut milk.

SUMMARY OF THE INVENTION

PROBLEMS TO BE SOLVED BY THE INVENTION

Plant milk has high nutritional value and has high value as a health food, and processing methods have been studied from various viewpoints. On the other hand, in view of further stably supplying plant milk, it cannot be said that control of the dispersion stability and/or solubility of the plant milk has been sufficiently studied. Techniques for improving dispersion stability and/or solubility have not been sufficiently studied not only in plant milk but also in various plant protein foods and drinks and materials thereof. In view of the possibility of spread and expansion of the plant protein food and drink and the material thereof in the future, a technique capable of improving the dispersion stability and/or solubility of the plant protein food and drink and the material thereof is desired in order to cope with diversification of their use applications.

An object of the present invention is to provide a processing technique of improving the dispersion stability and/or solubility of a plant protein food and drink and a material thereof.

MEANS FOR SOLVING THE PROBLEM

The present inventors have found that the dispersion stability and/or solubility of a plant protein food and drink and a material thereof can be enhanced by treating the plant protein food and drink and the material thereof with a protein deamidase, but there is still room for improvement in the effect, and it is considered that it is necessary to more efficiently exhibit the effect. Therefore, the present inventors have conducted intensive studies, and as a result, have found that the dispersion stability and/or solubility of a plant protein food and drink and a material thereof can be further enhanced by treating the plant protein food and drink and the material thereof with a combination of a protein deamidase and a lipase and/or a cyclodextrin glucanotransferase. That is, the present invention provides inventions of the following aspects.

Item 1. A production method for a processed product of a plant protein food and drink material and/or a plant protein food and drink, the production method including a step of treating a plant protein food and drink material and/or a plant protein food and drink with a protein deamidase and a lipase and/or a cyclodextrin glucanotransferase.

Item 2. The production method for processed plant milk described in item 1, in which the plant protein food and drink material and/or the plant protein food and drink is plant milk.

Item 3. The production method for processed plant milk described in item 1 or 2, in which the plant protein food and drink material and/or the plant protein food and drink is selected from the group consisting of oat milk, black bean milk, walnut milk, peanut milk, and coconut milk.

Item 4. The production method for processed plant milk described in any one of items 1 to 3, in which the plant protein food and drink material and/or the plant protein food and drink is walnut milk and/or peanut milk, and a lipase is used in the step.

Item 5. The production method for processed plant milk described in item 4, in which the lipase is a lipase derived from the genus Rhizopus or the genus Mucor.

Item 6. The production method for processed plant milk described in any one of items 1 to 3, in which the plant protein food and drink material and/or the plant protein food and drink is selected from the group consisting of oat milk, black bean milk, peanut milk, and coconut milk, and a cyclodextrin glucanotransferase is used in the step.

Item 7. The production method for processed plant milk described in item 3 or 6, in which a content of a coconut-derived component in the coconut milk is 10 to 70 w/v%.

Item 8. The production method for processed plant milk described in any one of items 1 to 7, in which the protein deamidase is used in an amount of 0.01 U or more per 1 g of a plant protein.

Item 9. The production method for processed plant milk described in any one of items 1 to 8, in which the lipase is used in an amount of 0.5 U or more per 1 g of a plant protein raw material.

Item 10. The production method for processed plant milk described in any one of items 1 to 9, in which the cyclodextrin glucanotransferase is used in an amount of 0.01 U or more per 1 g of a plant protein raw material.

Item 11. A dispersion stability improver for walnut milk and/or peanut milk, the dispersion stability improver containing a protein deamidase and a lipase.

Item 12. A dispersion stability improver for oat milk, the dispersion stability improver containing a protein deamidase and a cyclodextrin glucanotransferase.

Item 13. A solubility improver for plant milk selected from the group consisting of black bean milk, peanut milk, and coconut milk, the solubility improver containing a protein deamidase and a cyclodextrin glucanotransferase.

Item 14. A yield improver for coconut milk, the yield improver containing a protein deamidase and a cyclodextrin glucanotransferase.

ADVANTAGES OF THE INVENTION

According to the present invention, there is provided a processing technique of improving the dispersion stability and/or solubility of a plant protein food and drink and a material thereof.

EMBODIMENTS OF THE INVENTION

1. Production Method for Processed Product of Plant Protein Food and Drink Material and/or Plant Protein Food and Drink

A production method for a processed product of a plant protein food and drink material and/or a plant protein food and drink of the present invention includes a step of treating a plant protein food and drink material and/or a plant protein food and drink with a protein deamidase and a lipase and/or a cyclodextrin glucanotransferase. Hereinafter, the production method for a processed product of a plant protein food and drink material and/or a plant protein food and drink of the present invention will be described in detail.

1-1. Plant Protein Food and Drink Material and/or Plant Protein Food and Drink

The plant protein food and drink material and/or the plant protein food and drink used in the present invention is not particularly limited. The plant protein food and drink material refers to a food and drink material which contains a plant protein, is not directly eaten and/or drunk, but is premised on cooking, and is used as a food and drink material. The plant protein food and drink refers to a food and drink which is directly eaten and/or drunk. Specific examples of the plant protein food and drink material and/or the plant protein food and drink (hereinafter, these are collectively referred to as "plant protein food and drink material and the like") include plant milk, plant cream, plant substitute meat, plant substitute cheese, and a plant protein solution. Among these plant protein food and drink material and the like, from the viewpoint of further improving the effect of the present invention, those having fluidity such as plant milk, plant cream, and a plant protein solution are preferable, and plant milk is more preferable.

A plant edible part to be a raw material of the plant protein (hereinafter, described as "plant protein raw material") contained in the plant protein food and drink material and the like is not particularly limited, and examples thereof include cereals such as wheat and barley, rice, and beans, and nuts. Among these plants, from the viewpoint of further improving the effect of the present invention, oat, black bean, walnut, peanut, and coconut are preferable.

A specific method for preparing the plant protein food and drink material and the like using a plant protein raw material can be appropriately determined by those skilled in the art. For example, the amount of water per 1 part by weight of the plant protein raw material used for preparation of the plant protein food and drink material and the like is, for example, 0.5 to 40 parts by weight, 1 to 30 parts by weight, or 1.5 to 20 parts by weight.

A production method for processed plant milk of the present invention includes a step of treating plant milk with a protein deamidase and a lipase and/or a cyclodextrin glucanotransferase. Hereinafter, the production method for processed plant milk of the present invention will be specifically described.

The plant milk used in the present invention refers to a liquid in which a crushed product of a plant edible part is dispersed in water. The crushing of the plant edible part can be performed by any method such as squeezing and/or grinding, and these crushing methods can be preferably performed in water. In the plant milk, the crushed product of the edible part may be dispersed, and a component derived from the edible part exposed in water by extraction or the like may be partially or completely dissolved, dispersed, and/or emulsified. The plant milk may be obtained by removing an insoluble matter derived from the skin of the edible part or the like by any means such as centrifugal filtration, filtration, filtration bag, or sieve as appropriate, or may contain the insoluble matter in a state of being dispersed without the insoluble matter being removed.

The plant edible part to be a raw material of the plant milk used in the present invention is not particularly limited. In the present invention, preferably, the plant milk is selected from the group consisting of oat milk, black bean milk, walnut milk, peanut milk, and coconut milk.

The oat milk used in the present invention is not particularly limited, and general oat milk can be used. Examples of the oat milk include a liquid materials obtained by filtering a heat-treated oat slurry (for example, porridge of oat powder, a crushed product of oatmeal porridge, and the like). In the heat-treated oat slurry, the amount of water with respect to 1 part by weight of oat is, for example, 0.5 to 20 parts by weight, 1 to 15 parts by weight, or 2 to 10 parts by weight, preferably 3 to 8 parts by weight, more preferably 4 to 6 parts by weight, and further preferably 4.5 to 5.5 parts by weight. The temperature of the heating treatment of the oat slurry is, for example, 83 to 100°C, preferably 85 to 96°C, and more preferably 88 to 93°C. The number of meshes of a sieve used for filtering the heat-treated oat slurry may be such a degree that coarse insoluble fibers of oat are removed, and is, for example, 50 to 70 mesh and preferably 55 to 65 mesh.

The black bean milk used in the present invention is not particularly limited, and general black bean milk (black bean soy milk) can be used. Examples of the black bean milk include a heated black bean slurry (for example, a slurry product obtained by crushing heated-treated black bean with skin in water (preferably hot water)), a heat-treated product of the black bean slurry (for example, a heat-treated product of a slurry product obtained by crushing unheated black bean with skin in water), diluted products thereof, and pH-adjusted products thereof. The heating treatment temperature for the black bean or the slurry product is not particularly limited, and is, for example, 48°C to a boiling temperature °C. The temperature of the hot water is, for example, 80 to 95°C. The content of a black bean-derived component in the black bean milk is, for example, 0.5 to 25 w/v%, 1 to 20 w/v%, 2 to 15 w/v%, preferably 3 to 10 w/v%, more preferably 4 to 8 w/v%, and further preferably 5 to 7 w/v%. The content of a black bean protein in the black bean milk is, for example, 0.1 to 10 w/v%, 0.2 to 8 w/v%, 0.4 to 6 w/v%, 0.8 to 4 w/v%, preferably 1.2 to 3 w/v%, more preferably 1.5 to 2.5 w/v%, and further preferably 1.8 to 2.2 w/v%. The pH (25°C) of the black bean milk is, for example, 5.5 to 6.5 and preferably 5.8 to 6.2.

The walnut milk used in the present invention is not particularly limited, and general walnut milk can be used.

Examples of the walnut milk include a heat-treated walnut slurry (for example, a heat-treated product of a slurry of peeled walnut or a water-diluted product thereof). The temperature of the heating treatment of the walnut slurry is, for example, 83 to 100°C, preferably 85 to 96°C, and more preferably 88 to 93°C. The amount of water with respect to 1 part by weight of walnut in the walnut milk is, for example, 0.5 to 20 parts by weight, 1 to 15 parts by weight, 1.5 to 10 parts by weight, preferably 2 to 8 parts by weight, more preferably 3 to 5 parts by weight, and further preferably 3.5 to 4.5 parts by weight.

The peanut milk used in the present invention is not particularly limited, and general peanut milk can be used. Examples of the peanut milk include a heat-treated peanut slurry (for example, a boiled product of a slurry of peeled and roasted peanut or a water-diluted product thereof). The temperature of the heating treatment is, for example, 90°C to a boiling temperature, preferably 95°C to a boiling temperature, and more preferably a boiling temperature. The content of a peanut-derived component in the peanut milk is, for example, 0.5 to 25 w/v%, 1 to 20 w/v%, 2 to 15 w/v%, preferably 4 to 12 w/v%, more preferably 6 to 10 w/v%, further preferably 7 to 9 w/v%, and still more preferably 7.5 to 8.5 w/v%. The content of a peanut protein in the peanut milk is, for example, 0.1 to 10 w/v%, 0.2 to 8 w/v%, 0.4 to 6 w/v%, 0.8 to 4 w/v%, preferably 1.2 to 3 w/v%, more preferably 1.5 to 2.5 w/v%, and further preferably 1.8 to 2.2 w/v%. The pH (25°C) of the peanut milk is, for example, 5.5 to 6.5 and preferably 5.8 to 6.2.

The coconut milk used in the present invention is not particularly limited, and general coconut milk can be used. Examples of the coconut milk include a coconut slurry (for example, a slurry product obtained by pulverizing a crushed product and/or a minced product of endosperm or copra of the mature fruit of raw coconut (a dried product of endosperm of the mature fruit) in water (preferably in warm water) or a water-diluted product thereof). The temperature of the warm water is, for example, 40 to 60°C. The content of a coconut-derived component in the coconut milk is, for example, 10 to 70 w/v%, 20 to 60 w/v%, preferably 30 to 50 w/v%, and more preferably 35 to 45 w/v%, and the content of the protein in the coconut milk is, for example, 0.1 to 10 w/v%, 0.2 to 5 w/v%, 0.3 to 3 w/v%, preferably 0.5 to 2 w/v%, and further preferably 1 to 1.5 w/v%. The pH (25°C) of the coconut milk is, for example, 5.5 to 6.5 and preferably 5.8 to 6.2.

These kinds of plant milk may be used singly or in combination of a plurality of kinds thereof.

1-2. Protein Deamidase

The type, origin, and the like of the protein deamidase used in the present invention are not particularly limited as long as the protein deamidase is an enzyme that exhibits an action of decomposing an amide group-containing side chain of a protein without cleaving peptide bonds and crosslinking the protein. Examples of the protein deamidase include commercially available products of a protein deamidase derived from the genus Chryseobacterium, Flavobacterium, Empedobacter, Sphingobacterium, Aureobacterium, or Myroides, and a protein glutaminase derived from the genus Chryseobacterium, which are disclosed in JP 2000-50887 A, JP 2001-218590 A, and WO 2006/075772 A1. These protein deamidases may be used singly or in combination of a plurality of kinds thereof.

Among these protein deamidases, from the viewpoint of further improving the dispersion stability and/or solubility of the plant protein food and drink material and the like or the viewpoint of further improving the yield of coconut milk, a protein deamidase derived from the genus Chryseobacterium is preferable, a protein glutaminase derived from the genus Chryseobacterium is more preferable, and a protein glutaminase derived from Chryseobacterium proteolyticum is further preferable.

The protein deamidase can be prepared from a culture solution of a microorganism from which the protein deamidase is derived. Specific examples of the preparation method include a method of recovering a protein deamidase from a culture solution or a bacterial cell of the above-mentioned microorganism. For example, in the case of using a microorganism that secretes protein deamidase, an enzyme can be separated and/or purified after recovering bacterial cells from the culture solution in advance by filtration, a centrifugal treatment, or the like, as necessary. In the case of using a microorganism that does not secrete protein deamidase, an enzyme can be separated and/or purified after recovering bacterial cells from the culture solution in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include a centrifugal separation method, a UF concentration method, a salting-out method, and various chromatography methods using an ion-exchange resin or the like. The separated and/or purified enzyme can be powdered by a drying method such as freeze-drying or reduced-pressure drying and prepared as an enzymatic agent, and can also be powdered using an appropriate excipient and/or drying aid in the drying method.

As the protein deamidase, a commercially available enzymatic agent can also be used, and examples of a preferred commercially available product include a protein glutaminase "Amano" 500 manufactured by Amano Enzyme Inc.

The titer of the enzymatic agent containing the protein deamidase used in the present invention is not particularly limited, and is, for example, 10 to 50000 U, preferably 100 to 10000 U, more preferably 200 to 800 U/g, further preferably 300 to 700 U/g, still more preferably 400 to 600 U/g, and even more preferably 450 to 550 U/g.

The used amount of the protein deamidase is not particularly limited, but the used amount of the protein deamidase per 1 g of a plant protein in the plant protein food and drink material and the like is, for example, 0.01 U or more, preferably 0.1 U or more, more preferably 0.5 U or more, and further preferably 0.8 U or more and 25 U or less.

The used amount of the protein amidase per 1 g of the plant protein raw material used in the plant protein food and drink material and the like is, for example, 0.006 U or more, preferably 0.012 U or more, more preferably 0.024 U or more, further preferably 0.036 U or more, and still more preferably 0.06 U or more and 10 U or less.

When the plant protein food and drink material and the like are oat milk, from the viewpoint of further increasing the dispersion stability and/or solubility of the oat milk, preferably the viewpoint of further increasing dispersion stability, the used amount of the protein deamidase per 1 g of an oat protein is 0.1 U or more, 0.5 U or more, 1 U or more, preferably 1.5 U or more, more preferably 2 U or more, further preferably 2.5 U or more, still more preferably 3 U or more, even more preferably 4 U or more, and particularly preferably 4.5 U or more. The upper limit of the used amount range of the protein deamidase per 1 g of the oat protein is not particularly limited, and is, for example, 25 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of the oat protein is, for example, 22 U or less, preferably 17 U or less, more preferably 14 U or less, further preferably 10 U or less, still more preferably 8 U or less, and even more preferably 6 U or less.

When the plant protein food and drink material and the like are oat milk, the used amount of the protein deamidase per 1 g of oat used in the oat milk is, for example, 0.06 U or more, and from the viewpoint of further increasing the dispersion stability and/or solubility of the oat milk, preferably the viewpoint of further increasing dispersion stability, the used amount thereof is preferably 0.18 U or more, more preferably 0.24 U or more, further preferably 0.3 U or more, still more preferably 0.36 U or more, even more preferably 0.48 U or more, and particularly preferably 0.54 U or more. The upper limit of the used amount range of the protein deamidase per 1 g of oat is not particularly limited, and is, for example, 5 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of oat is, for example, 4 or less, preferably 3 U or less, more preferably 2 U or less, further preferably 1.5 U or less, still more preferably 1 U or less, and particularly preferably 0.7 U or less.

When the plant protein food and drink material and the like are black bean milk, from the viewpoint of further increasing the dispersion stability and/or solubility of the black bean milk, preferably the viewpoint of further increasing solubility (particularly, further increasing the amount of solute molecules that exhibit absorption at a wavelength of 280 nm), the used amount of the protein deamidase per 1 g of the black bean protein is 0.1 U or more, 0.5 U or more, preferably 1 U or more, more preferably 1.5 U or more, further preferably 2 U or more, and still more preferably 2.5 U or more. The upper limit of the used amount range of the protein deamidase per 1 g of the black bean protein is not particularly limited, and is, for example, 25 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of the black bean protein is, for example, 20 U or less, preferably 15 U or less, more preferably 10 U or less, further preferably 5 U or less, and still more preferably 3 U or less.

When the plant protein food and drink material and the like are black bean milk, the used amount of the protein deamidase per 1 g of black bean used in the black bean milk is, for example, 0.05 U or more, and from the viewpoint of further increasing the dispersion stability and/or solubility of the black bean milk, preferably the viewpoint of further increasing solubility (particularly, further increasing the amount of solute molecules that exhibit absorption at a wavelength of 280 nm), the used amount thereof is preferably 0.1 U or more, more preferably 0.25 U or more, further preferably 0.4 U or more, still more preferably 0.55 U or more, even more preferably 0.7 U or more, and particularly preferably 0.85 U or more. The upper limit of the used amount range of the protein deamidase per 1 g of black bean is not particularly limited, and is, for example, 10 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of black bean is, for example, 8 U or less, preferably 6 U or less, more preferably 4 U or less, further preferably 3 U or less, still more preferably 2 U or less, and even more preferably 1 U or less.

When the plant protein food and drink material and the like are walnut milk, from the viewpoint of further increasing the dispersion stability and/or solubility of the walnut milk, preferably the viewpoint of further increasing dispersion stability, the used amount of the protein deamidase per 1 g of a walnut protein is 0.1 U or more, 0.5 U or more, 1 U or more, preferably 1.5 U or more, more preferably 2 U or more, further preferably 2.5 U or more, still more preferably 3 U or more, even more preferably 4 U or more, and particularly preferably 5 U or more. The upper limit of the used amount range of the protein deamidase per 1 g of the walnut protein is not particularly limited, and is, for example, 25 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of the walnut protein is, for example, 22 U or less, preferably 17 U or less, more preferably 14 U or less, further preferably 12 U or less, still more preferably 9 U or less, even more preferably 7 U or less, and particularly preferably 6 U or less.

When the plant protein food and drink material and the like are walnut milk, the used amount of the protein deamidase per 1 g of walnut used in the walnut milk is, for example, 0.05 U or more, and from the viewpoint of further increasing the dispersion stability and/or solubility of the walnut milk, preferably the viewpoint of further increasing dispersion stability, the used amount thereof is preferably 0.1 U or more, more preferably 0.25 U or more, further preferably 0.4 U or more, still more preferably 0.55 U or more, even more preferably 0.7 U or more, and particularly preferably 0.85 U or more. The upper limit of the used amount range of the protein deamidase per 1 g of walnut is not particularly limited, and is, for example, 10 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of walnut is, for example, 8 U or less, preferably 6 U or less, more preferably 4 U or less, further preferably 3 U or less, still more preferably 2 U or less, and even more preferably 1.5 U or less.

When the plant protein food and drink material and the like are peanut milk, from the viewpoint of further increasing the dispersion stability and/or solubility of the peanut milk, preferably the viewpoint of further increasing solubility, the used amount of the protein deamidase per 1 g of the peanut protein is 0.05 U or more, preferably 0.1 U or more, more preferably 0.2 U or more, further preferably 0.4 U or more, still more preferably 0.6 U or more, even more preferably 0.8 U or more, and particularly preferably 0.9 U or more. The upper limit of the used amount range of the protein deamidase per 1 g of the peanut protein is not particularly limited, and is, for example, 25 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of the peanut protein is, for example, 20 U or less, preferably 15 U or less, more preferably 10 U or less, still more preferably 5 U or less, and even more preferably 3 U or less.

When the plant protein food and drink material and the like are peanut milk, the used amount of the protein deamidase per 1 g of peanut used in the peanut milk is, for example, 0.05 U or more, and from the viewpoint of further increasing the dispersion stability and/or solubility of the peanut milk, preferably the viewpoint of further increasing solubility, the used amount thereof is preferably 0.01 U or more, more preferably 0.05 U or more, further preferably 0.09 U or more, still more preferably 0.13 U or more, even more preferably 0.17 U or more, and particularly preferably 0.21 U or more. The upper limit of the used amount range of the protein deamidase per 1 g of peanut is not particularly limited, and is, for example, 10 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of peanut is, for example, 5 U or less, preferably 3 U or less, more preferably 2 U or less, further preferably 1 U or less, still more preferably 0.8 U or less, and even more preferably 0.7 U or less.

When the plant protein food and drink material and the like are coconut milk, from the viewpoint of further increasing the dispersion stability and/or solubility of the coconut milk of the protein deamidase, preferably the viewpoint of further increasing solubility (particularly, further increasing the amount of solute molecules that exhibit absorption at a wavelength of 280 nm), or the viewpoint of improving the yield of the coconut milk, the used amount of the protein deamidase per 1 g of the coconut protein is 0.1 U or more, 0.5 U or more, preferably 1 U or more, more preferably 1.5 U or more, further preferably 2 U or more, and still more preferably 2.5 U or more. The upper limit of the used amount range of the protein deamidase per 1 g of the coconut protein is not particularly limited, and is, for example, 25 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of the coconut protein is, for example, 20 U or less, preferably 15 U or less, more preferably 10 U or less, still more preferably 5 U or less, and even more preferably 3 U or less.

When the plant protein food and drink material and the like are coconut milk, the used amount of the protein deamidase per 1 g of coconut used in the coconut milk is, for example, 0.006 U or more, and from the viewpoint of further increasing the dispersion stability and/or solubility of the coconut milk, preferably the viewpoint of further increasing solubility (particularly, further increasing the amount of solute molecules that exhibit absorption at a wavelength of 280 nm), or the viewpoint of improving the yield of the coconut milk, the used amount thereof is preferably 0.012 U or more, more preferably 0.024 U or more, further preferably 0.036 U or more, still more preferably 0.06 U or more, and even more preferably 0.07 or more. The upper limit of the used amount range of the protein deamidase per 1 g of coconut is not particularly limited, and is preferably 0.6 U or less. Since the present invention is excellent in the effect of improving dispersion stability and/or solubility, the effect can be effectively obtained without using a large amount of the protein deamidase. From such a viewpoint, a suitable example of the upper limit of the used amount range of the protein deamidase per 1 g of coconut is, for example, 0.45 U or less, preferably 0.3 U or less, more preferably 0.15 U or less, and further preferably 0.09 U or less.

For the activity of the protein deamidase, benzyloxycarbonyl-L-glutaminylglycine (Z-Gln-Gly) is used as a substrate, and the amount of enzyme that liberates 1 μmol of ammonia per minute is defined as 1 unit (1 U).

1-3. Lipase

The type, origin, and the like of the lipase used in the present invention are not particularly limited as long as the lipase is an enzyme that hydrolyzes ester bonds constituting lipids, specifically, an enzyme that exhibits an activity of decomposing triglyceride and liberating fatty acid. Examples of the lipase include lipases derived from the genus Rhizopus such as Rhizopus delemar, Rhizopus oryzae, Rhizopus arrhizus, Rhizopus niveus, and Rhizopus javenicus; the genus Aspergillus such as Aspergillus niger; the genus Mucor such as Mucor javanicus and Mucor miehei; the genus Rhizomucor such as RhizoMucor miehei; the genus Thermomyces such as Thermomyces lanuginosus; the genus Pseudomonas, the genus Geotrichum; the genus Penicillium; and the genus Candida. These lipases may be used singly or in combination of a plurality of kinds thereof.

Among these lipases, from the viewpoint of further improving the dispersion stability and/or solubility of the plant milk, a 1,3-regiospecific lipase is preferable. Among these lipases, from the viewpoint of extremely remarkably improving the dispersion stability and/or solubility of the plant milk, lipases derived from the genus Rhizopus (preferably Rhizopus oryzae) and the genus Mucor (preferably Mucor javanicus) are particularly preferable. More specifically, among these lipases, from the viewpoint of extremely remarkably improving dispersion stability of plant milk, particularly, walnut milk, lipases derived from the genus Rhizopus (preferably Rhizopus oryzae) and the genus Mucor (preferably Mucor javanicus) are particularly preferable, and from the viewpoint of extremely remarkably improving solubility of plant milk, particularly, walnut milk and peanut milk (particularly, extremely remarkably increasing the amount of solute molecules that exhibit absorption at a wavelength of 280 nm), a lipase derived from the genus Rhizopus (preferably Rhizopus oryzae) is particularly preferable.

The lipase can be prepared from a culture solution of microorganisms from which the lipase is derived. The specific preparation method is the same as the method for preparing the protein deamidase described above.

As the lipase, a commercially available product can also be used, and examples of a preferred commercially available product include Lipase DF "Amano" 15 and Lipase MHA "Amano" 10SD manufactured by Amano Enzyme Inc.

In the present invention, preferable examples of the case of using a lipase include the case of treating walnut milk and/or peanut milk as the plant protein food and drink material and the like, and more preferable examples thereof include the case of using a lipase for the purpose of improving dispersion stability of walnut milk and/or the case of using a lipase for the purpose of improving solubility of peanut milk (particularly, increasing the amount of solute molecules that exhibit absorption at a wavelength of 280 nm).

The used amount of the lipase is not particularly limited, and the used amount of the lipase per 1 g of the plant protein raw material used in the plant protein food and drink material and the like is, for example, 0.5 U or more. From the viewpoint of further improving the dispersion stability and/or solubility of the plant milk, the used amount of the lipase per 1 g of the plant protein raw material used in the plant protein food and drink material and the like is preferably 1 U or more. In particular, in the case of using a lipase derived from the genus Rhizopus (preferably Rhizopus oryzae) as the lipase, the used amount of the lipase per 1 g of the plant protein raw material used in the plant protein food and drink material and the like is more preferably 10 U or more, further preferably 50 U or more, still more preferably 100 U or more, and even more preferably 140 U or more, from the viewpoint of further improving the dispersion stability and/or solubility of the plant milk, in the case of using a lipase derived from the genus Mucor as the lipase, the used amount of the lipase per 1 g of the plant protein raw material used in the plant protein food and drink material and the like is more preferably 1 U or more, further preferably 3 U or more, still more preferably 4 U or more, and even more preferably 5 U or more, from the viewpoint of further improving the dispersion stability and/or solubility of the plant milk.

The upper limit of the used amount range of the lipase per 1 g of the plant protein raw material used in the plant protein food and drink material and the like is not particularly limited, and is, for example, 1000 U or less, 800 U or less, or 550 U or less. In particular, in the case of using a lipase derived from the genus Rhizopus (preferably Rhizopus oryzae) as the lipase, since dispersion stability and/or solubility can be extremely efficiently improved, even when a small amount of the lipase is used, an excellent effect of improving dispersion stability and/or solubility can be obtained. From such a viewpoint, in the case of using a lipase derived from the genus Rhizopus (preferably Rhizopus oryzae) as the lipase, a suitable example of the upper limit of the used amount range of the lipase per 1 g of the plant protein raw material used in the plant protein food and drink material and the like is, for example, 1000 U or less, preferably 500 U or less, more preferably 300 U or less, and more preferably 200 U or less. In the case of using a lipase derived from the genus Mucor as the lipase, since dispersion stability and/or solubility, particularly, dispersion stability can be extremely efficiently improved, even when a small amount of the lipase is used, an excellent effect of improving dispersion stability and/or solubility, particularly, dispersion stability can be obtained. From such a viewpoint, in the case of using a lipase derived from the genus Mucor as the lipase, a suitable example of the upper limit of the used amount range of the lipase per 1 g of the plant protein raw material used in the plant protein food and drink material and the like is, for example, 50 U or less, preferably 30 U or less, more preferably 15 U or less, further preferably 9 U or less, and still more preferably 7 U or less.

The ratio of the used amount of the protein amidase and the used amount of the lipase is determined depending on the used amount of each enzyme, and from the viewpoint of further enhancing the effect of improving the dispersion stability and/or solubility of the plant protein food and drink material and the like, the ratio of the used amount of the lipase per 1 U of the protein deamidase is preferably 1 U or more. In the case of using a lipase derived from the genus Rhizopus (preferably Rhizopus oryzae) as the lipase, the ratio of the used amount of the lipase per 1 U of the protein deamidase is more preferably 10 U or more, further preferably 30 U or more, still more preferably 50 U or more, and even more preferably 70 U or more, from the viewpoint of further improving the dispersion stability and/or solubility of the plant milk. In the case of using a lipase derived from the genus Mucor as the lipase, the ratio of the used amount of the lipase per 1 U of the protein deamidase is more preferably 2 U or more and further preferably 5 U or more, from the viewpoint of further improving the dispersion stability and/or solubility of the plant milk.

The upper limit of the ratio range of the used amount of the lipase per 1 U of the protein deamidase is not particularly limited, and is, for example, 200 U or less. In the case of using a lipase derived from the genus Rhizopus (preferably Rhizopus oryzae) as the lipase, a suitable example of the upper limit of the ratio range of the used amount of the lipase per 1 U of the protein deamidase is preferably 100 U or less, more preferably 90 U or less, and further preferably 80 U or less, from the same viewpoint as described above. In the case of using a lipase derived from the genus Mucor as the lipase, a suitable example of the upper limit of the ratio range of the used amount of the lipase per 1 U of the protein deamidase is preferably 50 U or less, more preferably 30 U or less, further preferably 15 U or less, still more preferably 9 U or less, and even more preferably 7 U or less, from the same viewpoint as described above.

For the activity of the lipase, olive oil is used as a substrate, and the amount of enzyme that increases 1 micromolar of fatty acid per minute is defined as 1 unit (1 U).

1-4. Cyclodextrin Glucanotransferase

The type, origin, and the like of the cyclodextrin glucanotransferase used in the present invention are not particularly limited as long as the cyclodextrin glucanotransferase is an enzyme that acts on $\alpha$-1,4-glucan and produces cyclodextrin (CD), which is cyclic $\alpha$-1,4-glucan, by its intramolecular transfer activity. Examples of the cyclodextrin glucanotransferase include cyclodextrin glucanotransferases derived from the genus Bacillus such as Bacillus stearothermophilus, Bacillus megaterium, Bacillus circulans, Bacillus macerans, Bacillus ohbensis, and Bacillus clarkii; the genus klebsiella such as Klebsiella pneumoniae; the genus Thremoanaerobactor; and the genus Brevibacterium. These cyclodextrin glucanotransferases may be used singly or in combination of a plurality of kinds thereof.

Among these cyclodextrin glucanotransferases, from the viewpoint of further improving the dispersion stability and/or solubility of the plant protein food and drink material and the like, there is mentioned a cyclodextrin glucanotransferase preferably derived from the genus Bacillus, more preferably derived from the thermophilic genus Bacillus, that is, the genus Geobacillus, and further preferably derived from thermophilic Bacillus stearothermophilus, that is, Geobacillus stearothermophilus.

The cyclodextrin glucanotransferase can be prepared from a culture solution of microorganisms from which the cyclodextrin glucanotransferase is derived. The specific preparation method is the same as the method for preparing the protein deamidase described above.

As the cyclodextrin glucanotransferase, a commercially available product can also be used, and examples of a preferred commercially available product include Contizyme manufactured by Amano Enzyme Inc.

In the present invention, preferable examples of the case of using a cyclodextrin glucanotransferase include the case of treating oat milk, black bean milk, peanut milk, and/or coconut milk as the plant protein food and drink material and the like, and more preferable examples thereof include the case of using a cyclodextrin glucanotransferase for the purpose of improving dispersion stability of oat milk, the case of using a cyclodextrin glucanotransferase for the purpose of improving solubility of black bean milk (particularly, increasing the amount of solute molecules that exhibit absorption at a wavelength of 280 nm), the case of using a cyclodextrin glucanotransferase for the purpose of improving solubility of peanut milk (particularly, increasing the amount of lysate protein), and/or the case of using a cyclodextrin glucanotransferase for the purpose of solubility of coconut milk (particularly, increasing the amount of solute molecules that exhibit absorption at a wavelength of 280 nm) and/or improving the yield.

The used amount of the cyclodextrin glucanotransferase is not particularly limited, and the used amount of the lipase per 1 g of the plant protein raw material used in the plant protein food and drink material and the like is, for example, 0.01 U or more and 1000 U or less.

When the plant protein food and drink material and the like are oat milk, the used amount of the cyclodextrin glucanotransferase per 1 g of oat used in the oat milk is 0.01 U or more, and from the viewpoint of further increasing the dispersion stability and/or solubility of oat milk, preferably the viewpoint of further increasing dispersion stability, the used amount thereof is preferably 0.05 U or more, more preferably 0.1 U or more, further preferably 0.2 U or more, still more preferably 0.3 U or more, and even more preferably 0.4 U or more. Since the oat milk is milk containing particularly a large amount of starch among plant milk, there is a tendency that dispersion stability can be improved if a large amount of an α-amylase is used with the protein deamidase. On the other hand, since dispersion stability of the oat milk can be particularly efficiently improved by treating the oat milk with a protein deamidase in combination with a cyclodextrin glucanotransferase, an excellent dispersion stability-improving effect is obtained even when the used amount of the cyclodextrin glucanotransferase is relatively small. From such a viewpoint, a suitable example of the upper limit of the used amount range of the cyclodextrin glucanotransferase per 1 g of the plant protein raw material used in the plant protein food and drink material and the like is 2 U or less, preferably 1 U or less, more preferably 0.5 U or less, and further preferably 0.3 U or less.

When the plant protein food and drink material and the like are black bean milk, the used amount of the cyclodextrin glucanotransferase per 1 g of black bean used in the black bean milk is 0.01 U or more, and from the viewpoint of further increasing the dispersion stability and/or solubility of the black bean milk, preferably the viewpoint of further increasing solubility (particularly, further increasing the amount of solute molecules that exhibit absorption at a wavelength of 280 nm), the used amount thereof is preferably 0.05 U or more, more preferably 0.1 U or more, further preferably 0.2 U or more, still more preferably 0.3 U or more, and even more preferably 0.4 U or more. The upper limit of the used amount range of the cyclodextrin glucanotransferase per 1 g of black bean is not particularly limited, and a suitable example thereof is 10 U or less, 5 U or less, 3 U or less, 2 U or less, 1 U or less, or 0.5 U or less.

When the plant protein food and drink material and the like are peanut milk, the used amount of the cyclodextrin glucanotransferase per 1 g of peanut used in the peanut milk is, for example, 0.05 U or more, and from the viewpoint of further increasing the dispersion stability and/or solubility of the peanut milk, preferably the viewpoint of further increasing solubility, the used amount thereof is preferably 0.1 U or more, more preferably 1 U or more, further preferably 10 U or more, still more preferably 20 or more, even more preferably 30 U or more, and particularly preferably 35 U or more. The upper limit of the used amount range of the cyclodextrin glucanotransferase per 1 g of peanut is not particularly limited, and a suitable example thereof is 1000 U or less, 100 U or less, 80 U or less, 60 U or less, 50 U or less, or 45 U or less.

When the plant protein food and drink material and the like are coconut milk, the used amount of the cyclodextrin glucanotransferase per 1 g of coconut used in the coconut milk is, for example, 0.05 U or more, and from the viewpoint of further increasing the dispersion stability and/or solubility of the coconut milk, preferably the viewpoint of further increasing solubility, or the viewpoint of further increasing the yield of the coconut milk, the used amount thereof is preferably 0.1 U or more, more preferably 1 U or more, further preferably 10 U or more, still more preferably 20 or more, even more preferably 30 U or more, and particularly preferably 35 U or more. The upper limit of the used amount range of the cyclodextrin glucanotransferase per 1 g of coconut is not particularly limited, and a suitable example thereof is 1000 U or less, 100 U or less, 80 U or less, 60 U or less, 50 U or less, or 45 U or less.

The ratio of the used amount of the protein amidase and the used amount of the cyclodextrin glucanotransferase is determined depending on the used amount of each enzyme, and from the viewpoint of further enhancing the effect of improving the texture of the plant milk, the ratio of the used amount of the cyclodextrin glucanotransferase per 1 U of the protein deamidase is preferably 0.05 U or more and 1000 U or less.

When the plant protein food and drink material and the like are black bean milk, the ratio of the used amount of the cyclodextrin glucanotransferase per 1 U of the protein deamidase is preferably 0.1 U or more, more preferably 0.15 U or more, further preferably 0.3 U or more, and still more preferably 0.4 U or more, from the viewpoint of further increasing the dispersion stability and/or solubility of the black bean milk, preferably the viewpoint of further increasing solubility (particularly, further increasing the amount of solute molecules that exhibit absorption at a wavelength of 280 nm). When the plant protein food and drink material and the like are black bean milk, the upper limit of the ratio range of the used amount of the cyclodextrin glucanotransferase per 1 U of the protein deamidase is not particularly limited, and a suitable example thereof is 10 U or less, 5 U or less, 3 U or less, 2 U or less, 1 U or less, or 0.5 U or less.

When the plant protein food and drink material and the like are peanut milk, the ratio of the used amount of the cyclodextrin glucanotransferase per 1 U of the protein deamidase is preferably 1 U or more, more preferably 10 U or more, further preferably 30 U or more, still more preferably 50 U or more, and even more preferably 60 U or more, from the viewpoint of further increasing the dispersion stability and/or solubility of the peanut milk, preferably the viewpoint of further increasing solubility. When the plant protein food and drink material and the like are peanut milk, the upper limit of the ratio range of the used amount of the cyclodextrin glucanotransferase per 1 U of the protein deamidase is not particularly limited, and a suitable example thereof is 1000 U or less, 200 U or less, 150 U or less, 90 U or less, or 70 U or less.

When the plant protein food and drink material and the like are coconut milk, the ratio of the used amount of the cyclodextrin glucanotransferase per 1 U of the protein deamidase is preferably 10 U or more, preferably 100 U or more, more preferably 300 U or more, and further preferably 500 U or more, from the viewpoint of further increasing the dispersion stability and/or solubility of the coconut milk, preferably the viewpoint of further increasing solubility, or the viewpoint of further increasing the yield of the coconut milk. The upper limit of the ratio range of the used amount of the cyclodextrin glucanotransferase per 1 U of the protein deamidase is not particularly limited, and a suitable example thereof is 1000 U or less, 800 U or less, or 600 U or less.

For the activity of the cyclodextrin glucanotransferase, potato starch is used as a substrate, and the amount of enzyme that reduces the blue iodine color of starch by 1% per minute is defined as 1 unit (1 U).

1-5. Other Enzymes

In the production method of the present invention, when the plant protein food and drink material and the like are oat milk, from the viewpoint of further increasing the dispersion stability and/or solubility of the oat milk, preferably the viewpoint of further increasing dispersion stability, an $\alpha$-amylase can be used in combination with the above-described enzymes.

The origin of the $\alpha$-amylase is not particularly limited, and examples thereof include $\alpha$ amylases derived from the genus Aspergillus such as Aspergillus oryzae and Aspergillus niger; and the genus Bacillus such as Bacillus amyloliquefaciens, Bacillus subtilis, and Bacillus licheniformis, an $\alpha$-amylase derived from the genus Bacillus is preferable, and an $\alpha$-amylase derived from Bacillus amyloliquefaciens species is more preferable.

The used amount of the $\alpha$-amylase per 1 g of oat is, for example, 0.05 U or more, and preferably 0.1 U or more, 0.5 U or more, or 1 U or more. The upper limit of the used amount range of the $\alpha$-amylase is not particularly limited, and is, for example, 50 U or less. Since the present invention is excellent in the effect of improving the dispersion stability and/or solubility of oat milk, particularly, the effect of improving dispersibility, the effect can be effectively obtained without using a large amount of the $\alpha$-amylase that assists dispersion stability and/or solubility of oat milk. From such a viewpoint, a suitable example of the upper limit of the used amount range of the $\alpha$-amylase is, for example, 10 U or less, preferably 5 U or less, more preferably 4 U or less, further preferably 3 U or less, and still more preferably 2 U or less.

For the activity of the $\alpha$-amylase, soluble starch is used as a substrate, and the amount of enzyme that increases the reducing power corresponding to 10 mg of glucose per 30 minutes is defined as 1 unit (1 U).

1-6. Reaction Conditions, etc.

In the step of treating a plant protein food and drink material and the like with a protein deamidase and a lipase and/or a cyclodextrin glucanotransferase, by adding a protein deamidase, a lipase and/or a cyclodextrin glucanotransferase to the plant protein food and drink material and the like, and as necessary, adding, a lipase and/or a cyclodextrin glucanotransferase, and other enzymes to the plant protein food and drink material and the like, a composition of the plant protein food and drink material and the like containing the plant protein food and drink material and the like, the protein deamidase, and the lipase and/or the cyclodextrin glucanotransferase, or containing the plant protein food and drink material and the like, the protein deamidase, the lipase and/or the cyclodextrin glucanotransferase, and the other enzymes is prepared, and the composition of the plant protein food and drink material and the like is maintained in a heated state, so that the enzyme treatment reaction can be caused to proceed.

The heating temperature (enzyme treatment reaction temperature) of the composition of the plant protein food and drink material and the like is not particularly limited, and can be appropriately determined by those skilled in the art according to the optimal temperature of the enzyme to be used and/or thermal characteristics of the plant protein food and drink material and the like, etc., but is, for example, 40 to 70°C.

For example, when the plant protein food and drink material and the like are oat milk, the heating temperature of the oat milk composition is preferably 50 to 70°C, more preferably 55 to 65°C, and further preferably 58 to 62°C. When the plant protein food and drink material and the like are black bean milk, the heating temperature of the black bean milk composition is preferably 40 to 60°C, more preferably 45 to 55°C, and further preferably 48 to 52°C. When the plant protein food and drink material and the like are walnut milk, the heating temperature of the walnut milk composition is preferably 40 to 60°C, more preferably 45 to 55°C, and further preferably 48 to 52°C. When the plant protein food and drink material and the like are peanut milk, the heating temperature of the peanut milk composition is preferably 40 to 60°C, more preferably 45 to 55°C, and further preferably 48 to 52°C. When the plant protein food and drink material and the like are coconut milk, the heating temperature of the coconut milk composition is preferably 40 to 60°C, more preferably 45 to 55°C, and further preferably 48 to 52°C.

The enzyme treatment reaction time of the composition of the plant protein food and drink material and the like is not particularly limited, and may be appropriately determined according to the preparation scale of the composition, but is, for example, 0.5 hours or longer and preferably 1 hour or longer. The upper limit of the range of the enzyme treatment reaction time is not particularly limited, and is, for example, 24 hours or shorter, 12 hours or shorter, 8 hours or shorter, or 6 hours or shorter.

The enzyme treatment reaction can be terminated by an enzyme deactivation treatment with high heat. The enzyme deactivation treatment temperature is, for example, 85°C or higher and preferably 90°C or higher, and the enzyme deactivation treatment time is, for example, 5 to 25 minutes and preferably 10 to 20 minutes.

The composition of the plant protein food and drink material and the like after the end of the enzyme treatment is subjected to a post-treatment such as filtration as necessary to obtain processed plant milk. The number of meshes of a sieve used for filtering the composition of the plant protein food and drink material and the like after the end of the enzyme treatment is, for example, 80 to 120 mesh, preferably 85 to 115 mesh, more preferably 90 to 110 mesh, and further preferably 95 to 105 mesh.

Processed plant protein food and drink material and the like can be obtained as a food and drink material and the like with improved dispersion stability and/or dissolution stability as compared with the plant protein food and drink material and the like before the enzyme treatment. Specifically, processed oat milk can be obtained as milk with improved dispersion stability and/or dissolution stability, preferably improved dispersion stability as compared with oat milk before the enzyme treatment; processed black bean milk can be obtained as milk with improved dispersion stability and/or dissolution stability, preferably improved solubility (particularly, further increased amount of solute molecules that exhibit absorption at a wavelength of 280 nm) as compared with black bean milk before the enzyme treatment; processed walnut milk can be obtained as milk with improved dispersion stability and/or dissolution stability, preferably improved dispersion stability as compared with walnut milk before the enzyme treatment; processed peanut milk can be obtained as milk with dispersion stability and/or dissolution stability, preferably improved solubility as compared with peanut milk before the enzyme treatment; and processed coconut milk can be obtained as milk with improved dispersion stability and/or dissolution stability, preferably improved solubility (particularly, further increased amount of solute molecules that exhibit absorption at a wavelength of 280 nm) as compared with coconut milk before the enzyme treatment.

2. Use and Use Application of Enzymatic Agent

As described above, the combination of a protein deamidase and a lipase and/or a cyclodextrin glucanotransferase can improve the dispersion stability and/or solubility of the plant protein food and drink material. Therefore, the present invention also provides a use of an enzymatic agent containing a protein deamidase and a lipase for producing a dispersion stability improver for walnut milk and/or peanut milk; a use of an enzymatic agent containing a protein deamidase and a cyclodextrin glucanotransferase for producing a dispersion stability improver for oat milk; and a use of an enzymatic agent containing a protein deamidase and a cyclodextrin glucanotransferase for producing a solubility improver for plant milk selected from the group consisting of black bean milk, peanut milk, and coconut milk, and also provides a dispersion stability improver for walnut milk and/or peanut milk, the dispersion stability improver containing a protein deamidase and a lipase; a dispersion stability improver for oat milk, the dispersion stability improver containing a protein deamidase and a cyclodextrin glucanotransferase; and a solubility improver for plant milk selected from the group consisting of black bean milk, peanut milk, and coconut milk, the solubility improver containing a protein deamidase and a cyclodextrin glucanotransferase.

In the present invention, the improvement of the solubility of the plant protein food and drink material includes at least any one of an increase in the amount of solute molecules that exhibit absorption at a wavelength of 280 nm and an increase in the amount of lysate protein.

When the coconut milk is treated with a protein deamidase, solubility is improved; on the other hand, when the coconut milk composition after the end of the enzyme treatment is filtered to obtain processed coconut milk, the amount of the filtrate increases, so that the yield itself decreases. According to the present invention, by combining the cyclodextrin glucanotransferase with the protein deamidase, not only solubility is further improved, but also the amount of filtrate increased by the use of the protein deamidase can be reduced, and the yield of the processed coconut milk can be improved. Therefore, the present invention also provides a use of an enzymatic agent containing a protein deamidase and a cyclodextrin glucanotransferase for producing a yield improver for coconut milk, and a yield improver for coconut milk, the yield improver containing a protein deamidase and a cyclodextrin glucanotransferase.

The type, used amount, and the like of the component to be used in the use, the dispersion stability improver, the solubility improver, and the yield improver are as described in the section of "1. Production Method for Processed Plant Milk".

EXAMPLES

Hereinafter, the present invention will be specifically described by means of Examples; however, the present invention is not to be construed as being limited to the following Examples.

Used Enzyme

Details of enzymes used in the following test examples are as follows.

[Table 1]

| Enzyme type | Abbreviation | Trade name | Origin |
|---|---|---|---|
| Protein glutaminase | PG | Protein-glutaminase | Chryseobacterium proteolyticum |
| α-Amylase | E5NC | Kleistase E5NC | Bacillus amyloliquefaciens |
| α-Amylase | BZ-LC | Biozyme LC | Aspergillus oryzae |
| β-Amylase | BAF | β-Amylase F | Bacillus flexus |
| Transglucosidase | TGL | Transglucosidase L | Aspergillus niger |
| Lipase | LDF | Lipase DF15 | Rhizopus oryzae |
| Lipase | LM | Lipase MHA10SD | Mucor javanicus |
| Cyclodextrin glucanotransferase | CGT | Contizyme | Geobacillus stearothermophilu |

The activity of the protein deamidase (protein glutaminase) was measured by the following method.

(1) To 1 ml of a 0.2 M phosphate buffer (pH 6.5) containing 30 mM Z-Gln-Gly, 0.1 ml of an aqueous solution containing a protein deamidase was added, the mixture was incubated at 37°C for 10 minutes, and then 1 ml of a 0.4 M TCA solution was added to stop the reaction. As a blank, to 1 ml of a 0.2 M phosphate buffer (pH 6.5) containing 30 mM Z-Gln-Gly, 1 ml of a 0.4 M TCA solution was added, 0.1 ml of an aqueous solution containing a protein deamidase was further added, and the mixture was incubated at 37°C for 10 minutes.

(2) The amount of ammonia generated in the reaction solution was measured for the solution obtained in (1) using Ammonia Test Wako (Wako Pure Chemical Industries, Ltd.). The ammonia concentration in the reaction solution was determined from a calibration curve representing the relationship between the ammonia concentration and the absorbance (630 nm) prepared using an ammonia standard solution (ammonium chloride).

(3) The activity of the protein deamidase was calculated from the following formula with the amount of enzyme that produces 1 μmol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

[Mathematical Formula 1]

Enzyme activity (U/mL)

= Ammonia concentration in reaction solution (mg/L) × (1/17.03) × (Reaction solution

amount/Enzyme solution amount) × (1/10) × Df

The activity of the lipase was measured by the following method.

In a container of an emulsifier, 75 mL of olive oil and 225 mL of an emulsion (polyvinyl alcohol I test solution or polyvinyl alcohol I-polyvinyl alcohol II test solution) were placed, and the mixture was stirred and emulsified intermittently at 14500 rotations per minute for 10 minutes (rotation for 3 minutes and 20 seconds → stop for 3 minutes and 20 seconds -> rotation for 3 minutes and 20 seconds -> stop for 3 minutes and 20 seconds -> rotation for 3 minutes and 20 seconds) while being cooled to 10°C or lower to obtain a substrate solution. This substrate solution was left to stand in a cold place (5 to 10°C) for 1 hour, and used after confirming that the oil layer was not separated. To 5 mL of the substrate solution, 4 mL of a buffer solution (phosphate buffer solution (0.1 mol/L) having a pH of 7.0) was added and shaken, the mixture was warmed at 37°C for 10 minutes, 1 mL of a sample solution was then added and immediately shaken, and the mixture was warmed at 37°C for 20 minutes. To this solution, 10 mL of an ethanol (95)/acetone mixed solution (1/1 at a volume ratio) was added and shaken, 10 mL of a 0.05 mol/L sodium hydroxide solution was then added, and 10 mL of the ethanol (95)/acetone mixed solution (1/1 at a volume ratio) was further added and shaken to obtain a test solution. Separately, 4 mL of the same buffer solution as in the case of the test solution was added to 5 mL of a substrate solution and shaken, the mixture was warmed at 37°C for 30 minutes, 10 mL of an ethanol (95)/acetone mixed solution (1/1 at a volume ratio) was then added, 1 mL of a sample solution was added and shaken, 10 mL of a 0.05 mol/L sodium hydroxide solution was added, and 10 mL of the ethanol (95)/acetone mixed solution (1/1 at a volume ratio) was further added and shaken to obtain a comparative solution. Two drops of a phenolphthalein test solution as an indicator were added to the test solution and the comparative solution, and immediately, while nitrogen gas was blown onto the liquid surface, the solution was titrated with 0.05 mol/L hydrochloric acid to pH 10.0. Under the present conditions, the amount of enzyme that increases 1 micromole of fatty acid per minute was defined as 1 unit (1 U), and the activity of the lipase was calculated by the following formula.

[Mathematical Formula 2]

$$\text{Enzyme activity (U/g, U/mL)} = 50 \times (T0 - T30) / 30 \times f \times n$$

T0: Titration amount (mL) of comparative solution
T30: Titration amount (mL) of test solution
50: Fatty acid equivalent amount (micromole) with respect to 1 mL of 0.05 mol/L hydrochloric acid (for quantitative determination)
30: Reaction time (min)
f: Factor of 0.05 mol/L hydrochloric acid
n: Dilution factor per 1 g of sample

The activity of the cyclodextrin glucanotransferase was measured by the following method.

1.0 g of potato starch was weighed, 20 mL of water was added, and 5 mL of a sodium hydroxide test solution (1 mol/L) was gradually added while stirring to form a paste. The paste was heated in a boiling water bath for 3 minutes with stirring, and then 25 mL of water was added. After cooling, the pH was adjusted to 5.5 with an acetic acid test solution (1 mol/L), and water was added to make 100 mL, thereby obtaining a substrate solution. 10 mL of the substrate solution was weighed and warmed at 40°C for 10 minutes, 1 mL of a sample solution was added and immediately shaken, the mixture was warmed at 40°C for 10 minutes, and 1 mL of this solution was weighed, added to 10 mL of a hydrochloric acid test solution (0.1 mol/L), and immediately shaken. 1 mL of this solution was weighed, and 10 mL of an iodine-potassium iodide test solution (0.4 mmol/L) was added and shaken to obtain a test solution. Separately, the same operation as in the preparation of the test solution was performed using water instead of the sample solution to prepare a comparative solution. For the test solution and the comparative solution, the absorbance at a wavelength of 660 nm was measured. Under the present conditions, the amount of enzyme that reduces the blue iodine color of starch by 1% per minute was defined as 1 unit (1 U).

[Mathematical Formula 3]

$$\text{Enzyme activity(U/g)} = (A0 - A10) / A0 \times 100 / 10 \times n$$

A10: Absorbance of reaction solution
A0: Absorbance of blank solution
100: Conversion factor of %
10: Reaction time (min)
n: Dilution factor per 1 g or 1 mL of sample

Evaluation of Dispersion Stability and Solubility

The dispersion stability and solubility of processed plant milk prepared in the following test examples were evaluated by an instability index by LUMiSizer 651, A280, a soluble protein concentration, and the like. The instability index by LUMiSizer 651 is an index based on the behavior of particles during centrifugation, and is used as an evaluation index of "dispersion stability". A280 represents the total amount of solute molecules that exhibit absorption at a wavelength of 280 nm, for example, proteins having aromatic amino acid (tyrosine, tryptophan) residues, and is used as an evaluation index of "solubility". The soluble protein concentration represents a value measured by the Lowry method, that is, the amount of a protein having a tyrosine residue, a tryptophan residue, and/or a cysteine residue, and is used as an evaluation index of "solubility". Details of the measurement conditions and the like of each evaluation item will be described in each test example.

Test Example 1

(1) Preparation of Black Bean Milk

To 208.5 g of black bean, 1300 mL of water was added, and the mixture was kept warm in an electric oven (50°C) for 4 hours, then heated to 100°C with a cooking stove, and boiled for about 5 seconds. 160 mL of hot water at 90°C was further added, and the mixture was treated with a colloid mill for 40 minutes to prepare a black bean slurry. The black bean slurry was cooled to room temperature, 2000 mL of water was added, and the pH was adjusted to 6.0 (25°C) using 0.5 M citric acid. Water was added to the black bean slurry to give a volume of 3750 mL, thereby obtaining black bean milk (pH 6.0, 25°C) having a black bean-derived component content of 5.56 w/v% and a protein content of 2 w/v%. The obtained black bean milk was parceled out by 500 ml into a beaker under stirring.

(2) Enzyme Treatment

The enzymes shown in Table 2 were charged into 500 ml of the black bean milk in the indicated amounts, and reacted at 50°C for 6 hours. After performing an enzyme deactivation treatment at 90°C for 15 minutes, the mixture was stirred with a mixer for 5 minute to obtain processed black bean milk.

(3) Evaluation

The pH (25°C) and A280 of the processed black bean milk thus obtained were measured. The results are shown in Table 2.

<A280>

The processed black bean milk was centrifuged at 16000 rpm for 10 minutes, the supernatant was filtered through a 0.45 μm membrane filter, and the absorbance at 280 nm was measured.

[Table 2]

|  | Comparative Example | | Example |
| --- | --- | --- | --- |
|  | 1-1 | 1-2 | 1 |
| PG (U/1 g black bean protein) | 0 | 2.5 | 2.5 |
| CGT (U/1 g black bean) | 0 | 0 | 0.4 |
| pH | 6.24 | 6.46 | 6.39 |
| A280 | 0.472 | 0.519 | 0.590 |

As is apparent from Table 2, from the comparison between Comparative Example 1-1 and Comparative Example 1-2, it was found that the solubility of the black bean milk was improved by using the protein glutaminase, but as shown in Example 1, the solubility of the black bean milk was particularly remarkably improved by using the cyclodextrin glucanotransferase in combination with the protein glutaminase.

Test Example 2

(1) Preparation of Walnut Milk

In a 1 w/w% sodium hydroxide solution, 350 g of walnut was immersed, and heated (70°C or higher) and stirred for 10 minutes. The sodium hydroxide solution was discarded, and the skin was peeled off, and the walnut was washed with water and drained. To 300 g of the obtained peeled walnut, 1000 ml of hot water (80°C) was added, and the mixture was treated with a colloid mill for 30 minutes to prepare a walnut slurry. The walnut slurry was heated at 90°C for 15 minutes and then cooled to 50°C, and warm water was added to give a volume of 1500 g, thereby preparing walnut milk (the total amount of water with respect to 1 part by weight of peeled walnut was 4 parts by weight). The prepared walnut milk was parceled out under stirring.

(2) Enzyme Treatment

The enzymes shown in Table 3 were charged in the indicated amounts, and reacted at 50°C for 3 hours. After performing an enzyme deactivation treatment at 90°C for 15 minutes, the mixture was stirred and filtered through a sieve (100 mesh) to obtain processed walnut milk.

(3) Evaluation

A280 and the instability index of the processed walnut milk thus obtained were measured. The results are shown in Table 3.

<A280>

The processed walnut milk was centrifuged at 14000 rpm for 10 minutes, the supernatant was diluted 50 times, and the absorbance at 280 nm was measured.

instability Index>

The instability index was measured using LUMiSizer 651 under the conditions of 4000 rpm (RCA 2100 g), 25°C, 865 nm, 300 profiles, Interval 10s, and light factor 1.

[Table 3]

| | Comparative Example | | | Example | |
|---|---|---|---|---|---|
| | 2-1 | 2-2 | 2-3 | 2-1 | 2-2 |
| PG (U/1 g walnut protein) | 0 | 11 | 5.5 | 11 | 5.5 |
| LDF (U/1 g walnut) | 0 | 0 | 0 | 150 | 0 |
| LM (U/1 g walnut) | 0 | 0 | 0 | 0 | 6.5 |
| A280 | 0.45 | 0.63 | 3.1 | 0.76 | 3.2 |
| Instability index | 0.41 | 0.16 | 0.41 | 0.05 | 0.01 |
| *11 U of PG per 1 g of walnut protein corresponds to 2 U per 1 g of walnut. | | | | | |

As is apparent from Table 3, from the comparison of Comparative Example 2-1, Comparative Example 2-2, and Comparative Example 2-3, it was found that the solubility and dispersion stability of the walnut milk were improved by using the protein glutaminase, but as shown in Example 2-1 and Example 2-2, the dispersion stability of the walnut milk was particularly remarkably improved by using the lipase in combination with the protein glutaminase. As shown in Example 2-1, by using the lipase derived from the genus Rhizopus in combination with the protein glutaminase, the solubility of the walnut milk was also further improved.

Test Example 3

(1) Preparation of Peanut Milk

In 350 g of shelled peanut, 1400 mL (0.8 w/v%) of salt water was put, and the mixture was subjected to a boiling treatment for 5 minutes. The mixture was cooled and drained to remove a foreign material (skin and the like). The peeled peanut was roasted at 150°C for 40 minutes in an electric oven and cooled to remove a foreign material. 300 g of the obtained peanut was weighed, 1800 mL of warm water (50°C) was added, and the mixture was treated with a colloid mill for 40 minutes to obtain a peanut slurry. To the peanut slurry, 1700 mL of water was added, the mixture was boiled for 5 minutes, cooled to room temperature, adjusted to pH 6.0 (0.5 M citric acid), and warm water was added to give a volume of 3750 mL. As a result, peanut milk (pH 6.0) having a peanut-derived component content of 8 w/v% and a protein content of 2 w/v% was prepared. The prepared peanut milk was parceled out by 500 ml under stirring.

(2) Enzyme Treatment

The enzymes shown in Table 4 were charged in the indicated amounts, and reacted at 50°C for 1 hour, 3 hours, or 6 hours. After performing an enzyme deactivation treatment at 90°C for 15 minutes, the mixture was stirred and filtered through a sieve (100 mesh) to obtain processed peanut milk.

(3) Evaluation

The pH (25°C), the soluble protein concentration (mg/mL), and the protein solubilization rate (%) of the processed peanut milk thus obtained were measured. The results are shown in Table 4.

<Soluble Protein Concentration>

The processed peanut milk was centrifuged (16000xg, 10 minutes), and the soluble protein concentration was measured by the Lowry method using the supernatant filtrate (0.45 $\mu$m filter).

<Protein Solubilization Rate>

The ratio (wt%) of the soluble protein when the total protein of the processed peanut milk was regarded as 100 wt% was determined.

[Table 4]

|  | Comparative Example | | Example |
|---|---|---|---|
|  | 3-1 | 3-2 | 3 |
| PG (U/1 g peanut protein) | 0 | 2.5 | 2.5 |
| CGT (U/1 g peanut) | 0 | 0 | 40 |
| pH | 6.46 | 6.59 | 6.51 |
| Soluble protein concentration (mg/mL) | 3.3 | 5.3 | 6.3 |
| Protein solubilization rate (%) | 17 | 26 | 32 |

As is apparent from Table 4, from the comparison between Comparative Example 3-1 and Comparative Example 3-2, it was found that the solubility of the peanut milk was improved by using the protein glutaminase, but as shown in Example 3, the solubility of the peanut milk was remarkably improved by using the cyclodextrin glucanotransferase in combination with the protein glutaminase.

Test Example 4

(1) Preparation of Coconut Milk

1500 g of coconut meat (endosperm of the mature coconut fruit) was cut into 1 cm square with a knife, 2000 mL of warm water (50°C) was added thereto, and the mixture was treated with a colloid mill for 120 minutes while the filtrate was separated with a 100-mesh filter to prepare a coconut slurry. The separated filtrate was returned to the coconut slurry, the pH was adjusted to 6.0 (25°C) with 0.5 M citric acid, and then warm water (50°C) was added to give a volume of 3750 mL, thereby preparing coconut milk having a coconut-derived component content of 40 w/v% and a protein content of 1.2 w/v%. The prepared coconut milk was parceled out by 500 ml under stirring.

(2) Enzyme Treatment

The enzymes shown in Table 5 were charged into 500 ml of the coconut milk in the indicated amounts, and reacted at 50°C for 6 hours. After performing an enzyme deactivation treatment at 90°C for 15 minutes, the mixture was stirred with a mixer for 5 minute and filtered through a filter (100-mesh) to obtain processed coconut milk.

(3) Evaluation

The pH (25°C), A280, and the yield (%) of the processed coconut milk thus obtained were measured. The results are shown in Table 5.

<A280>

The processed coconut milk was centrifuged at 16000 g for 10 minutes, the supernatant was diluted 100 times, and the absorbance at 280 nm was measured.

< Yield>

When the volume of 500 ml of the coconut milk composition after the enzyme deactivation treatment in the above (2) was regarded as 100%, the relative volume (%) of the processed coconut milk after removing the filtrate of the coconut milk composition with a filter (100-mesh) was derived as a yield.

[Table 5]

|  | Comparative Example | | Example |
| --- | --- | --- | --- |
|  | 4-1 | 4-2 | 4 |
| PG (U/1 g coconut protein) | 0 | 2.5 | 2.5 |
| CGT (U/1 g coconut) | 0 | 0 | 40 |
| pH | 5.82 | 6.31 | 5.94 |
| A280 | 0.251 | 0.341 | 0.401 |
| Yield (%) | 64 | 58 | 66 |
| *2.5 U of PG per 1 g of coconut protein corresponds to 0.075 U per 1 g of coconut. | | | |

As is apparent from Table 5, from the comparison between Comparative Example 4-1 and Comparative Example 4-2, it was found that the solubility of the coconut milk was improved by using the protein glutaminase, but the amount of loss as a filtrate increased, and thus the yield decreased. On the other hand, as shown in Example 4, by using the cyclodextrin glucanotransferase in combination with the protein glutaminase, not only the solubility of the coconut milk was further improved, but also the yield was particularly remarkably improved.

Test Example 5

It also confirmed that, when oat milk (the total amount of water with respect to 1 part by weight of oat was about 5 parts by weight) was treated by adding the protein glutaminase and the cyclodextrin glucanotransferase, the solubility of the oat milk was improved, and when the peanut milk prepared in Test Example 3 was treated by adding the protein glutaminase and the lipase, the solubility of the peanut milk was improved.

**Claims**

1. A production method for a processed product of a plant protein food and drink material and/or a plant protein food and drink, the production method comprising a step of treating a plant protein food and drink material and/or a plant protein food and drink with a protein deamidase and at least one enzyme selected from the group consisting of a lipase and a cyclodextrin glucanotransferase.

2. The production method according to claim 1, wherein the plant protein food and drink material and/or the plant protein food and drink is plant milk.

3. The production method according to claim 1, wherein the plant protein food and drink material and/or the plant protein food and drink is selected from the group consisting of oat milk, black bean milk, walnut milk, peanut milk, and coconut milk.

4. The production method according to claim 1, wherein the plant protein food and drink material and/or the plant protein food and drink is walnut milk and/or peanut milk, and a lipase is used in the step.

5. The production method according to claim 4, wherein the lipase is selected from the group consisting of lipases derived from the genus Rhizopus and the genus Mucor.

6. The production method according to claim 1, wherein the plant protein food and drink material and/or the plant protein food and drink is selected from the group consisting of oat milk, black bean milk, peanut milk, and coconut milk, and a cyclodextrin glucanotransferase is used in the step.

7. The production method according to claim 2, wherein a content of a coconut-derived component in the coconut milk is 10 to 70 w/v%.

8. The production method according to claim 1, wherein the protein deamidase is used in an amount of 0.01 U or more per 1 g of a plant protein.

9. The production method according to claim 1, wherein the lipase is used in an amount of 0.5 U or more per 1 g of a plant protein raw material.

10. The production method according to claim 1, wherein the cyclodextrin glucanotransferase is used in an amount of 0.01 U or more per 1 g of a plant protein raw material.

11. A dispersion stability improver for walnut milk and/or peanut milk, the dispersion stability improver comprising a protein deamidase and a lipase.

12. A dispersion stability improver for oat milk, the dispersion stability improver comprising a protein deamidase and a cyclodextrin glucanotransferase.

13. A solubility improver for plant milk selected from the group consisting of black bean milk, peanut milk, and coconut milk, the solubility improver comprising a protein deamidase and a cyclodextrin glucanotransferase.

14. A yield improver for coconut milk, the yield improver comprising a protein deamidase and a cyclodextrin glucanotransferase.

## INTERNATIONAL SEARCH REPORT

| International application No. |
| --- |
| **PCT/JP2021/040557** |

| A. CLASSIFICATION OF SUBJECT MATTER |
| --- |
| *A23J 3/14*(2006.01)i; *A23J 3/34*(2006.01)i; *A23C 11/10*(2021.01)i; *A23L 11/60*(2021.01)i; *A23L 11/65*(2021.01)i; *A23L 2/66*(2006.01)i; *A23L 2/38*(2021.01)i <br> FI: A23J3/34; A23C11/10; A23L2/00 J; A23L2/38 D; A23J3/14 |
| According to International Patent Classification (IPC) or to both national classification and IPC |

| B. FIELDS SEARCHED |
| --- |
| Minimum documentation searched (classification system followed by classification symbols) <br> A23J3/14; A23J3/34; A23C11/10; A23L11/60; A23L11/65; A23L2/66; A23L2/38 |
| Documentation searched other than minimum documentation to the extent that such documents are included in the fields searched <br><br> Published examined utility model applications of Japan 1922-1996 <br> Published unexamined utility model applications of Japan 1971-2021 <br> Registered utility model specifications of Japan 1996-2021 <br> Published registered utility model applications of Japan 1994-2021 |
| Electronic data base consulted during the international search (name of data base and, where practicable, search terms used) <br><br> CAplus/MEDLINE/EMBASE/BIOSIS/CABA/FSTA (STN) |

| C. DOCUMENTS CONSIDERED TO BE RELEVANT |
| --- |

| Category* | Citation of document, with indication, where appropriate, of the relevant passages | Relevant to claim No. |
| --- | --- | --- |
| X | WO 2012/117879 A1 (AMANO ENZYME INC.) 07 September 2012 (2012-09-07) <br> claims 1-8, examples 9, 10 | 1, 8, 9 |
| A | claims 1-8, examples 9, 10 | 2-5, 7, 11 |
| X | JP 2000-513231 A (NOVO NORDISK A/S) 10 October 2000 (2000-10-10) <br> claims 1-17 | 1, 8, 9 |
| A | claims 1-17 | 2-5, 11 |
| A | JP 2016-506732 A (OATLY AB) 07 March 2016 (2016-03-07) <br> Claims 1-23, examples | 1-5, 7-9, 11 |
| A | WO 2017/009100 A1 (DSM IP ASSETS B. V.) 19 January 2017 (2017-01-19) <br> claims 1-12, examples 3, 5 | 1-5, 7-9, 11 |
| A | WO 2017/154992 A1 (AJINOMOTO KK) 14 September 2017 (2017-09-14) <br> claims 1-13, examples | 1-5, 7-9, 11 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search <br><br> **24 December 2021** | Date of mailing of the international search report <br><br> **11 January 2022** |
| --- | --- |
| Name and mailing address of the ISA/JP <br><br> **Japan Patent Office (ISA/JP)** <br> **3-4-3 Kasumigaseki, Chiyoda-ku, Tokyo 100-8915** <br> **Japan** | Authorized officer <br><br><br><br> Telephone No. |

Form PCT/ISA/210 (second sheet) (January 2015)

## INTERNATIONAL SEARCH REPORT

International application No.

**PCT/JP2021/040557**

### C. DOCUMENTS CONSIDERED TO BE RELEVANT

| Category* | Citation of document, with indication, where appropriate, of the relevant passages | Relevant to claim No. |
|---|---|---|
| A | JP 2016-502868 A (IMPOSSIBLE FOODS INC.) 01 February 2016 (2016-02-01)<br>claims 1-102, examples | 1-5, 7-9, 11 |
| A | JP 2015-216846 A (T HASEGAWA CO., LTD.) 07 December 2015 (2015-12-07)<br>claims 1-9 | 1-5, 7-9, 11 |
| A | WO 2020/171105 A1 (AMANO ENZYME INC.) 27 August 2020 (2020-08-27)<br>claims 1-17 | 1-14 |
| A | WO 2020/171106 A1 (AMANO ENZYME INC.) 27 August 2020 (2020-08-27)<br>claims 1-20 | 1-14 |
| A | WO 2020/176469 A1 (AMANO ENZYME USA CO., LTD.) 03 September 2020 (2020-09-03)<br>claims 1-39 | 1-14 |
| A | 荒勝俊ら, 豆乳の食味に及ぼす酵素処理の影響, 日本食品科学工学会誌, 50(1), 2003, 13-21. (Journal of the Japanese Society for Food Science and Technology), non-official translation (ARA, Katsutoshi et al., Enzyme Treatment Influence on Soy Milk Taste)<br>entire text | 1-3, 6-8, 10, 12-14 |
| A | JP 2012-34654 A (T HASEGAWA CO., LTD.) 23 February 2012 (2012-02-23)<br>entire text | 1-3, 6-8, 10, 12-14 |
| A | JP 11-318373 A (KAO CORP.) 24 November 1999 (1999-11-24)<br>entire text | 1-3, 6-8, 10, 12-14 |
| A | JP 6-98704 A (RHONE-POULENC INC.) 12 April 1994 (1994-04-12)<br>entire text | 1-3, 6-8, 10, 12-14 |

Form PCT/ISA/210 (second sheet) (January 2015)

## INTERNATIONAL SEARCH REPORT

International application No.

**PCT/JP2021/040557**

**Box No. III**    **Observations where unity of invention is lacking (Continuation of item 3 of first sheet)**

This International Searching Authority found multiple inventions in this international application, as follows:

Document 1: WO 2012/117879 A1 (AMANO ENZYME INC.) 07 September 2012 (2012-09-07)
Document 2: JP 2000-513231 A (NOVO NORDISK A/S) 10 October 2000 (2000-10-10)
Document 3: JP 2016-506732 A (OATLY AB) 07 March 2016 (2016-03-07)
Document 4: WO 2017/009100 A1 (DSM IP ASSETS B.V.) 19 January 2017 (2017-01-19)

Document 1 (in particular, claims 1-8, examples 9, 10), document 2 (in particular, claims 1-17), document 3 (in particular, claims 1-23, examples), and document 4 (in particular, claims 1-12, examples 3, 5) can be said to disclose various types of methods for manufacturing a processed article of a vegetable protein food/drink material and/or a vegetable protein food/drink, the methods including a step for processing a vegetable protein food/drink material and/or a vegetable protein food/drink with protein deamidase and other types of enzymes. In particular, documents 1 and 2 also indicate that lipase is used as the other enzyme.

Claim 1 sets forth a method for manufacturing a processed article of a vegetable protein food/drink material and/or a vegetable protein food/drink, the method including a step for processing a vegetable protein food/drink material and/or a vegetable protein food/drink with at least one enzyme selected from the group consisting of protein deamidase, lipase, and cyclomaltodextrin glucanotransferase. Claim 1 thus includes a case in which protein deamidase and lipase are used together as enzymes, and a case in which protein deamidase and cyclomaltodextrin glucanotransferase are used together as enzymes. As described above, using various types of enzymes together, such as protein deamidase and lipase as enzymes, was well known prior to the filing of the present application in light of documents 1-4, and thus this feature cannot be said to be a special technical feature.

Furthermore, lipase and cyclomaltodextrin glucanotransferase have different catalyst substrates and reactions, and can also not be said to be technically linked enzymes.

Therefore, claim 1 includes a section having the special technical feature of "processing with protein deamidase and lipase" as enzymes, and a section having the special technical feature of "processing with protein deamidase and cyclomaltodextrin glucanotransferase" as enzymes.

(Invention 1) Claims 1-5, 7-9, 11

As described above, the section of claim 1 having the special technical feature of "processing with protein deamidase and lipase" as enzymes and claims 2-5 and 7-9 citing this section are classified as invention 1.

Additionally, claim 11 is has a feature of "including protein deamidase and lipase" and thus can be said to share the same or corresponding feature with the section of claim 1 having the special technical feature of "processing with protein deamidase and lipase" as enzymes. Therefore, claim 11 is also classified as invention 1.

(Claim 2) Claims 1-3, 6-8, 10, 12-14

As described above, the section of claim 1 having the special technical feature of "processing with protein deamidase and cyclomaltodextrin glucanotransferase" as enzymes and claims 2-3, 6-8, and 10 citing this section are classified as invention 2.

Additionally, claims 12-14 have a feature of "including protein deamidase and cyclomaltodextrin glucanotransferase" and can be said to share the same or corresponding feature with the section of claim 1 having the special technical feature of "processing with deamidase and cyclomaltodextrin glucanotransferas" as enzymes. Therefore, claims 12-14 are also classified as invention 2.

Form PCT/ISA/210 (continuation of first sheet) (January 2015)

## INTERNATIONAL SEARCH REPORT

International application No.

**PCT/JP2021/040557**

| Box No. III | Observations where unity of invention is lacking (Continuation of item 3 of first sheet) |

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☑ No protest accompanied the payment of additional search fees.

Form PCT/ISA/210 (continuation of first sheet) (January 2015)

**INTERNATIONAL SEARCH REPORT**
**Information on patent family members**

International application No.

**PCT/JP2021/040557**

| Patent document cited in search report | | | Publication date (day/month/year) | Patent family member(s) | | | Publication date (day/month/year) |
|---|---|---|---|---|---|---|---|
| WO | 2012/117879 | A1 | 07 September 2012 | US | 2014/0037790 | A1 | |
| | | | | claims 1-8, examples 9, 10 | | | |
| | | | | CN | 103402362 | A | |
| JP | 2000-513231 | A | 10 October 2000 | WO | 1998/000029 | A1 | |
| | | | | claims 1-17 | | | |
| | | | | EP | 912100 | A1 | |
| JP | 2016-506732 | A | 07 March 2016 | WO | 2014/123466 | A1 | |
| | | | | claims 1-23, examples | | | |
| | | | | US | 2015/0351432 | A1 | |
| | | | | EP | 2953482 | A1 | |
| | | | | KR | 10-2015-0113200 | A | |
| | | | | CN | 105007757 | A | |
| WO | 2017/009100 | A1 | 19 January 2017 | EP | 3322305 | A1 | |
| WO | 2017/154992 | A1 | 14 September 2017 | US | 2019/0037883 | A1 | |
| | | | | claims 1-13, examples | | | |
| | | | | EP | 3427595 | A1 | |
| JP | 2016-502868 | A | 01 February 2016 | WO | 2014/110540 | A1 | |
| | | | | claims 1-102, examples | | | |
| | | | | US | 2015/0305361 | A1 | |
| | | | | US | 2015/0305390 | A1 | |
| | | | | US | 2015/0351435 | A1 | |
| | | | | EP | 2943077 | A1 | |
| | | | | EP | 3513664 | A1 | |
| | | | | CN | 105050422 | A | |
| | | | | KR | 10-2015-0105979 | A | |
| JP | 2015-216846 | A | 07 December 2015 | (Family: none) | | | |
| WO | 2020/171105 | A1 | 27 August 2020 | (Family: none) | | | |
| WO | 2020/171106 | A1 | 27 August 2020 | (Family: none) | | | |
| WO | 2020/176469 | A1 | 03 September 2020 | (Family: none) | | | |
| JP | 2012-34654 | A | 23 February 2012 | (Family: none) | | | |
| JP | 11-318373 | A | 24 November 1999 | (Family: none) | | | |
| JP | 6-98704 | A | 12 April 1994 | EP | 577294 | A2 | |
| | | | | whole document | | | |
| | | | | US | 5380542 | A | |
| | | | | US | 5294456 | A | |
| | | | | US | 5294457 | A | |
| | | | | US | 5585131 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000050887 A
- JP 2008283900 A
- JP 2015159765 A
- US 6451361 B1
- CN 101991163 A
- JP 2006230297 A
- WO 2019104971 A1
- JP 2008099676 A
- JP 2001218590 A
- WO 2006075772 A1